# FASCICULE DE BREVET EUROPEEN

(11) **EP 1 082 333 B1**
(45) Date de publication et mention de la délivrance du brevet: **18.09.2002**
(21) Numéro de dépôt: 99918043.3
(22) Date de dépôt: 11.05.1999
(51) Int. Cl.: C07H 15/248, A61K 31/70

(54) **DERIVE DE THIOCOLCHICOSIDE, SA PREPARATION ET SON APPLICATION EN THERAPEUTIQUE**
THIOCOLCHICOSID-DERIVATE, IHRE HERSTELLUNG UND VERWENDUNG ALS ARZNEIMITTEL
THIOCOLCHICOSIDE DERIVATIVE, PREPARATION AND THERAPEUTIC APPLICATION

(30) Priorité: 27.05.1998 FR 9806677
(43) Date de publication de la demande: 14.03.2001
(73) Titulaire: SANOFI-SYNTHELABO, 75013 Paris (FR)
(72) Inventeur: THENOT, Jean-Paul, F-91190 Gif sur Yvette (FR); ESPIE, Pascal, F-92350 Le Plessis Robinson (FR); ALLEN, John, F-78180 Montigny le Bretonneux (FR); DEPOORTERE, Henri, F-78720 Cernay la Ville (FR)
(74) Mandataire: Thouret-Lemaitre, Elisabeth
(86) Numéro de dépôt international: FR9901114
(87) Numéro de publication internationale: WO99061457

(56) Documents cités:
- EP-A- 0 789 028
- WO-A-96/11184
- DE-A- 2 220 796
- FR-A- 2 112 131

## Description

La présente invention a pour objet le composé répondant à la formule (I) du schéma en annexe.

Le composé est un dérivé du thiocolchicoside (ou 2-deméthoxy-2-glucosidoxy-thiocolchicine) composé bien connu pour son activité myorelaxante et décontracturante. (voir EP-A-789 028)

Le composé (I) a été préparé selon le schéma réactionnel joint en annexe et l'exemple ci-dessous.
1 - Dans un premier temps on prépare le composé (III) à partir du dérivé triacétylé de l'ester méthylique de l'acide β-D-glucuronique de formule 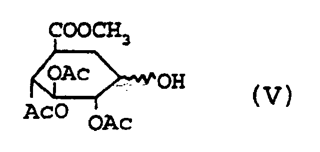
   Dans un ballon tricol sous argon et sous agitation mécanique, on introduit 9,65 g (28,8 mmole) du composé (V), 15,2 ml de thichloroacétonitrile dans 150 ml de dichlorométhane. Après 15 mn on ajoute 3,98 g de carbonate de potassium finement broyé. On agite pendant 20 heures puis on filtre sur un fritté rempli de silice et mouillé à l'éther, on élue 6 fois avec de l'éther et on évapore à sec.
   On reprend le résidu dans de l'isopropanol. Une cristallisation lente s'amorce. Après refroidissement on filtre le composé (8,3 g).
2 - On hydrolyse le thiocolchicoside (4,7 g = 8,34 mmole) par action d'acide chlorhydrique (100 ml) à la température du reflux et sous agitation. Après filtration du précipité et lavage 3 fois à l'eau, puis 2 fois au toluène, on le sèche. Après chromatographie sur gel de silice par le mélange chloroforme 95/méthanol 5, on recueille 2,9 g de composé (II).
3 - Dans un ballon tricol, sous argon et agitation magnétique, on introduit 1,4 g (3,48 mmole) de composé (II), 130 ml de dichlorométhane, 3,34 g de composé (III) et 0,89 ml de composé BF₃ O(C₂H₅)₂.

On porte le mélange à la température du reflux pendant 4 heures puis on le laisse revenir à la température ambiante. On y ajoute de la glace puis de l'eau, on laisse décanter, et on extrait le résidu au dichlorométhane. On le lave à l'eau, le sèche sur sulfate de sodium, le filtre et l'évapore à sec. Après chromatographie sur gel de silice par l'éluant mélange éther 92/méthanol 8 puis éther 90/méthanol 10, on obtient 3,05 g de composé (IV).

On hydrolyse le composé (IV) (2,35 g = 3,27 mmole) dans 100 ml de méthanol par addition, goutte à goutte, à l'aide d'une ampoule à brome, de 13,1 ml d'hydroxyde de sodium 1N.

On agite le mélange à la température ambiante pendant 4 heures, puis on ajoute de la glace et de l'eau, on amène le pH à 3 avec de l'acide chlorhydrique 1N, on extrait le résidu 3 fois avec du butanol et on évapore à sec. Après chromatographie sur silice par l'éluant chloroforme 70/méthanol 30 et 1 % d'acide acétique, on obtient 3,4 g de composé (I). Le composé fond à 240-242°C.

L'activité décontraccurante du composé de l'invention a été déterminée par l'inhibition du réflexe polysynaptique chez le rat normal (RPRN). L'animal est placé en contention en décubitus ventral. Les membres postérieurs sont immobilisés. Les réflexes polysynaptiques sont évoqués dans le muscle Biceps fémoral (fléchisseur de la cuisse) par stimulation électrique de la voûte plantaire (territoire cutané du nerf sural). L'EMG (Electro-Myo-Graphie) est recueilli par deux électrodes en acier inoxydable insérées dans le muscle parallélement aux fibres.

Les électrodes de stimulation en acier inoxydable sont insérées en position sous-cutanée. La stimulation consiste en un choc électrique carré de 0.2-0.4 ms de durée et de 0.2 Hz de fréquence. L'intensité de la stimulation est réglée pour obtenir des réflexes bien constitués et de surface stable (8-12 mA).

L'EMG est amplifié. La surface de chaque réflexe est mesurée.

Des séries de 20 réflexes sont enregistrées toutes les 15 minutes. La surface des 20 réflexes de chaque série est moyennée.

Après plusieurs séries contrôles, le produit à étudier est administré. La moyenne des 20 premières réponses contrôles est prise pour 100 %. L'évolution des réponses EMG est suivie alors pendant 2 ou 3 heures.

Le composé de l'invention à la dose de 10 mg/kg par voie i.p inhibe le réflexe polysynaptique de 78 %.

Le composé de l'invention peut être utilisé sous la forme de composé, gélule, capsule ou solution pour l'administration par voie orale, parentérale, ou intramusculaire, à raison de 8 à 32 mg par jour en plusieurs prises ou injections.

Le composé de l'invention peut être utilisé pour le traitement des contractures musculaires douloureuses au cours des affections vertébrales (torticolis, dorsalgies, lombalgies) et pour le traitement des contractures neurologiques avec spasticité.

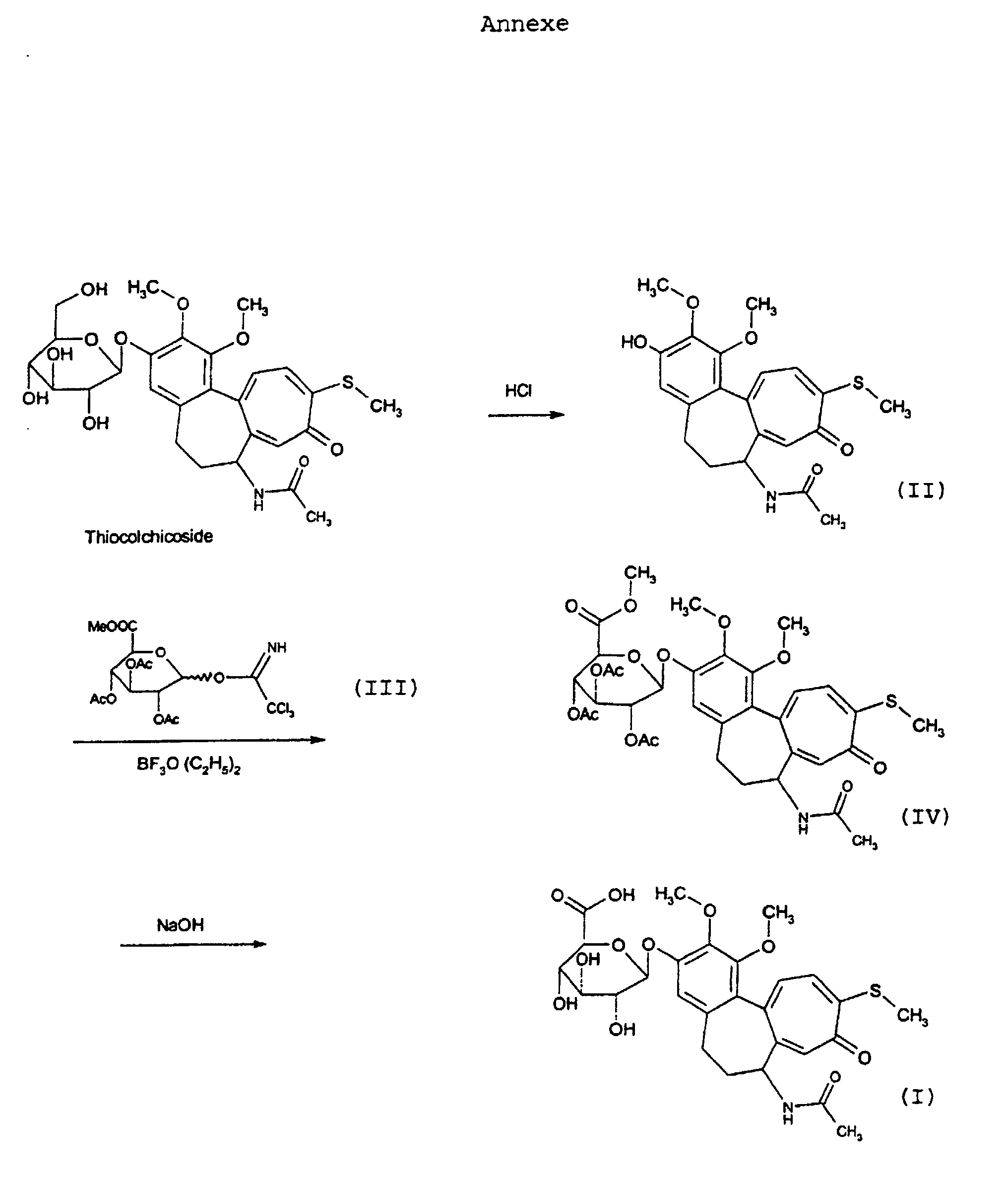

## Revendications

1. Dérivé de thiocolchicoside répondant à la formule (I)

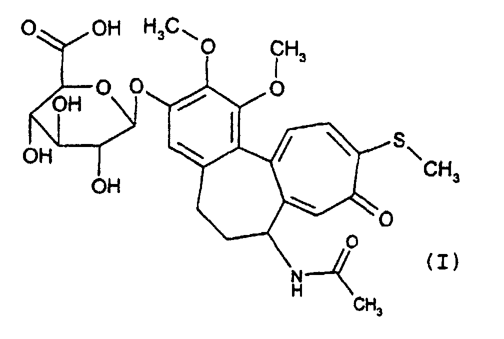

2. Procédé de préparation du dérivé de la revendication 1, **caractérisé en ce que** l'on fait réagir le composé de formule (II) 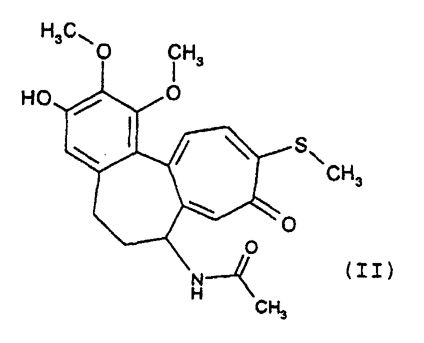 obtenu par hydrolyse du thiocolchicoside avec le composé de formule (III) 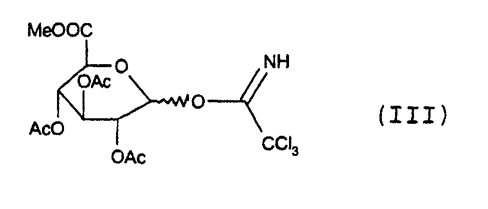 puis on hydrolyse le composé obtenu de formule (IV)

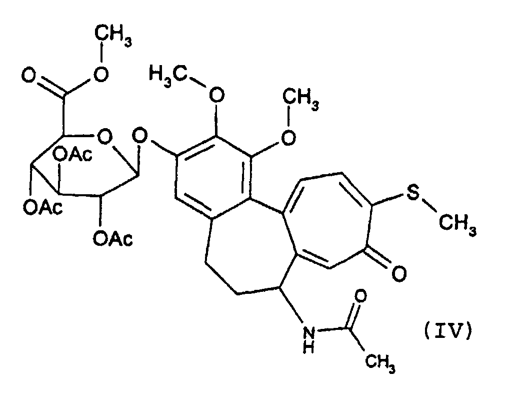

3. Médicament **caractérisé en ce qu'**il contient le composé de la revendication 1.

4. Composition pharmaceutique **caractérisée en ce qu'**elle contient le composé de la revendication 1 en association avec tout excipient approprié pour l'administration orale, parentérale ou intramusculaire.

## Claims

1. Thiocolchicoside derivative corresponding to the formula (I)

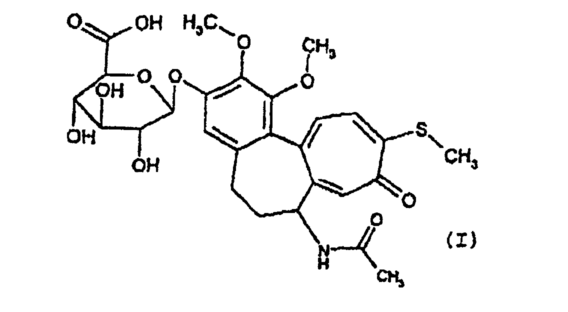

2. Method for preparing the derivative of Claim 1, **characterized in that** the compound of formula (II) 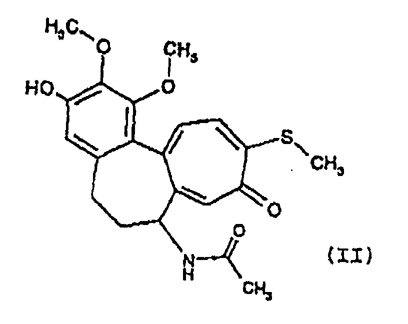 obtained by hydrolysis of thiocolchicoside, is reacted with the compound of formula (III) 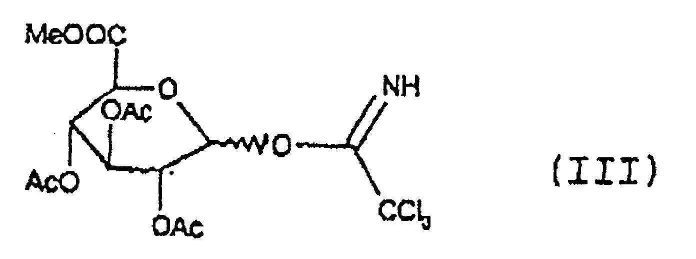 and then the compound obtained of formula (IV) 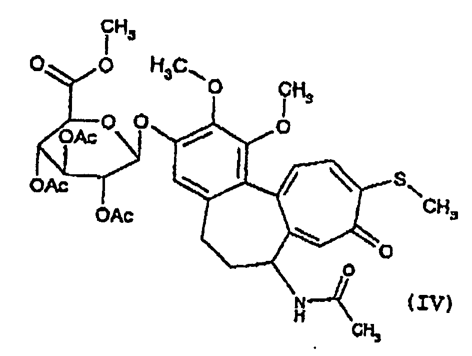 is hydrolysed.

3. Medicament, **characterized in that** it contains the compound of Claim 1.

4. Pharmaceutical composition, **characterized in that** it contains the compound of Claim 1 in combination with any appropriate excipient for oral, parenteral or intramuscular administration.

## Patentansprüche

1. Thiocolchicosid-Derivat der Formel (I):

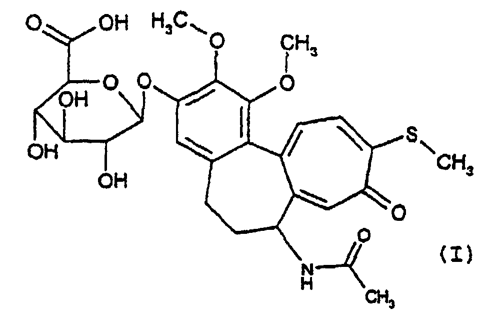

2. Verfahren zur Herstellung des Derivats nach Anspruch 1, **dadurch gekennzeichnet, daß** man die Verbindung der Formel (II): 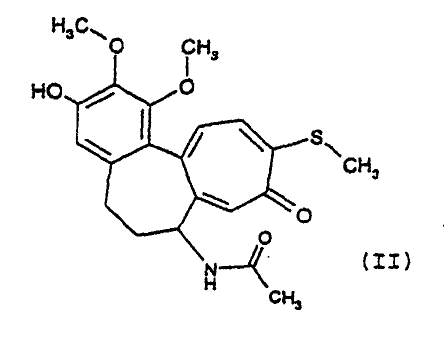 die durch Hydrolyse von Thicolchicosid erhalten worden ist, mit der Verbindung der Formel (III): 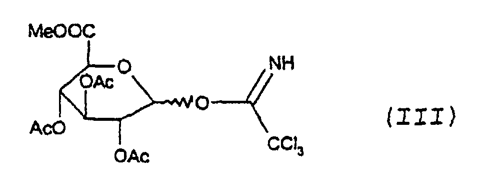 umsetzt und dann die erhaltene Verbindung der Formel (IV): 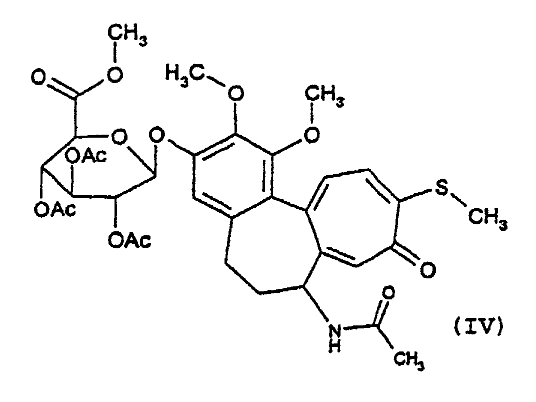 hydrolysiert.

3. Arzneimittel, **dadurch gekennzeichnet, daß** sie die Verbindung nach Anspruch 1 enthält.

4. Pharmazeutische Zubereitung, **dadurch gekennzeichnet, daß** sie die Verbindung nach Anspruch 1 in Kombination mit irgendeinem für die Verabreichung auf oralem, parenteralem und intramuskulärem Wege geeigneten Trägermaterial enthält.
